# EUROPEAN PATENT SPECIFICATION

(11) **EP 3 744 246 B1**
(45) Date of publication and mention of the grant of the patent: **28.04.2021**
(21) Application number: 19177227.6
(22) Date of filing: 29.05.2019
(51) Int. Cl.: A61B 5/11, A61B 5/00, A61B 50/13, A61G 12/00

(54) **MULTIDIMENSIONAL TOOL FOR ASSESSING THE RISK OF FALLING IN OLDER PEOPLE**
MEHRDIMENSIONALES WERKZEUG ZUR BEWERTUNG DER STURZGEFAHR BEI ÄLTEREN MENSCHEN
OUTIL MULTIDIMENSIONNEL POUR ÉVALUER LE RISQUE DE CHUTE CHEZ LES PERSONNES ÂGÉES

(30) Priority: 27.05.2019 PT 2019115541
(43) Date of publication of application: 02.12.2020
(73) Proprietor: Universidade de Évora, 7000-803 Évora (PT)
(72) Inventor: ROSA PARREIRA MENDES, FELISMINA, 1000-117 Lisboa (PT); LINO NETO PEREIRA, CATARINA, 7000-741 Évora (PT); DUARTE DOS SANTOS BRAVO, JORGE, 7005-638 Évora (PT); DE CASTRO MARTINS SECCA RUIVO, MARIA INÊS, 7000-585 Évora (PT); DA MOTA CERVEIRA NAZULINO GONÇALVES, TIAGO JOSÉ, 7000-536 Évora (PT)
(74) Representative: Ferreira, Maria Silvina

(56) References cited:
- Anonymous: "Custom Medical Cases | Cases By Source", , 12 September 2017 (2017-09-12), XP055620378, Retrieved from the Internet: URL:https://web.archive.org/web/2017091202 2147/https://www.casesbysource.com/categor y/custom-medical-cases [retrieved on 2019-09-10]
- HERNANDEZ D ET AL: "Predicting Which Older Adults Will or Will Not Fall Using the Fullerton Advanced Balance Scale", ARCHIVES OF PHYSICAL MEDICINE AND REHABILITATION, W.B. SAUNDERS, UNITED STATES, vol. 89, no. 12, 1 December 2008 (2008-12-01), pages 2309-2315, XP025740569, ISSN: 0003-9993, DOI: 10.1016/J.APMR.2008.05.020 [retrieved on 2008-11-01]
- NGUYEN HUNG M ET AL: "Differences in Physical Fitness and Subjectively Rated Physical Health in Vietnamese and German Older Adults", JOURNAL OF CROSS-CULTURAL GERONTOLOGY, SPRINGER US, BOSTON, vol. 28, no. 2, 11 May 2013 (2013-05-11), pages 181-194, XP035321894, ISSN: 0169-3816, DOI: 10.1007/S10823-013-9195-4 [retrieved on 2013-05-11]
- PEI YING SIM ET AL: "A Comparison Study of Portable Foot-to-Foot Bioelectrical Impedance Scale to Measure Body Fat Percentage in Asian Adults and Children", BIOMED RESEARCH INTERNATIONAL, vol. 2014, 1 January 2014 (2014-01-01), pages 1-10, XP055620392, ISSN: 2314-6133, DOI: 10.1155/2014/475659

## Description

### Technical domain

The present application concerns a tool for assessing the risk of falling in older people.

### Background

Falls are one of the main causes of health problems in older people who live in the community and are responsible for two-thirds of all unintentional injuries that lead to death, being the main cause of potentially preventable injuries. Thirty per cent of community-dwelling people aged 65 years old or more fall at least once a year and 15% fall more than once. Falls directly or indirectly reduce mobility, physical activity and physical functioning, and are often caused by the fear of falling again, which paradoxically increases the likelihood of another fall, reducing mobility and quality of life.
Because falls are a serious and costly health problem, the accurate assessing of the risk of falling is a critical component of the health assessment of older people who live in the community. National and international health authorities recommend a multifactorial risk assessment of the risk of falling that includes valid and reliable tools, scientifically tested on populations with specific socio-demographic and cultural characteristics, allowing for sufficient individualization of the programs to prevent falls based on the physical and cognitive state of the older person. It is intended that these tools should demonstrate efficiency and precision in the identification of older people at risk of falling.
In order for a fall risk assessment tool to meet the current requirements of the health authorities, it should allow for a multifactorial assessment, including the assessment of several fall risk factors simultaneously, such as the assessment of balance, of the strength of the lower limbs, of impaired walking, the history of falls, the environmental hazards, habitual medication, and the main health problems and cognitive deficits. The recommendations also require that the tool should be able to be administered by informal caregivers with specific technical training and should not be exclusive to formal researchers and/or caregivers, and also allow for a single and continuous final score, which permits the repeated verification of the risk of falling in the same person, enabling a clear sense of the reduction or worsening of the individual risk of falling.
Currently, both nationally and internationally, there is no single tool that combines perfectly validated and reliable instruments, which allows for the assessment of the risk of falls in older people in accordance with the criteria recommended by the aforementioned health authorities.
The current methods of diagnosing the risk of falls in the independent older seek to evaluate, in isolation, various determinants of the risk of falling, whether clinical, morphological, functional or cognitive, giving them an individual discriminatory power. These methods overlook the holistic perspective of the life of the human being, where the various determinants are combined into a single system, interacting dynamically, with each, nevertheless, assuming its preponderance in the individual system of the older person.
In fact, currently there only exist elements that separately assess the various risk factors for falls, but which are not combined in a single product, which prevents the large-scale application of diagnostic tests by formal and informal caregivers, and limits the opportunity to compare the results of various populational niches, mainly due to the difficulty of ensuring the reliable replication of the diagnostic tests. Document "https://www.casesbysource.com/category/custom-medical-cases" (D1), is a website disclosing medical cases that can be customizable with an assortment of medical tools, that might comprise several storage levels, and is described as a packing solution. Document D1 does not disclose a multidimensional tool specifically developed as a solution for assessing the risk of falling in older people, it does not disclose a trolley box for easy transportation of instruments, and wherein said toolbox is itself an instrument adapted for the realization of tests to evaluate the strength of the lower limbs, agility and dynamic balance of older people, as is the case of the present multidimensional tool. Document "Predicting which older adults will or will not fall using the Fullerton Advanced Balance Scale, Hernandez, D. et al., (2008)" (D2), is a study article that specifically assessed if the Fullerton Advanced Balance Scale (FAB) can predict faller status in a group of independently functionally older adults. Document D2 does not disclose a multidimensional tool specifically developed as a solution for assessing the risk of falling in older people, it does not disclose a trolley box for easy transportation of instruments, and wherein said toolbox is itself an instrument adapted for the realization of tests to evaluate the strength of the lower limbs, agility and dynamic balance of older people, as is the case of the present multidimensional tool. Furthermore, the FAB test is just one of the many tests that can be performed with the presently disclosed tool.

Document "Differences in physical fitness and subjectivity rated physical health in Vietnamese and German older adults, Nguyen, H. et al., (2013)" (D3) is a study article that investigates the differences in physical fitness in two specific communities. Document D3 does not disclose a multidimensional tool specifically developed as a solution for assessing the risk of falling in older people, it does not disclose a trolley box for easy transportation of instruments, and wherein said toolbox is itself an instrument adapted for the realization of tests to evaluate the strength of the lower limbs, agility and dynamic balance of older people, as is the case of the present multidimensional tool. The tool herein disclosed is also adapted to be used in different communities.

Document "A comparison study of portable foot-to-foot bioelectrical impedance scale to measure body fat percentage in Asian adults and children, Sim, P. et al., (2014) is a study article that aims to test a specific instrument, the bioimpedance scale. Document D4 does not disclose a multidimensional tool specifically developed as a solution for assessing the risk of falling in older people, it does not disclose a trolley box for easy transportation of instruments, and wherein said toolbox is itself an instrument adapted for the realization of tests to evaluate the strength of the lower limbs, agility and dynamic balance of older people, as is the case of the present multidimensional tool. The bioimpedance scale is only one of several instruments in the disclosed tool. Furthermore, D4 does not disclose an alternative dynamic model to obtain body composition data as a substitute to the bioimpedance scale.

### Summary

The invention is defined by a multidimensional tool according to claim **1**. Embodiments are disclosed by dependent claims **2-12.** This application concerns a multidimensional tool (1) for evaluating the risk of falling in the older people consisting of a trolley comprising two consecutive levels of instruments, where:
- The first level of the tool (1) comprises digital stopwatches (2), a bioimpedance scale (12), a metronome (13), mattresses (14), an instruction manual and digital medium with the manual (16);
- The second level of the tool (1) comprises a 150cm anthropometrical tape (3), a 20m measuring tape (4), flat markers (5), retractable cones 30cm in height (6), a 30cm ruler (7), a 50cm ruler (8), a stadiometer (9), threaded dumbbell handles (11), threaded dumbbell weights (10), a means of writing (15).

The tool has dimensions of 150mm in height, 360mm in width and 460mm in length.
In one embodiment the tool comprises four retractable cones (6) .
In another embodiment the tool comprises four dumbbell weights (10).
In another embodiment the tool comprises two dumbbell handles (11) .
In yet another embodiment each dumbbell (10) weighs 0.68 Kg.

In another embodiment each dumbbell handle (11) weighs 0.91 Kg.

In one embodiment the tool comprises ten flat markers (5).

In another embodiment the tool comprises two digital stopwatches (2).

In one embodiment the tool comprises a support (17) for the flat markers (5) and the retractable cones (6).

In one embodiment the tool comprises two mattresses (14).

In another embodiment the means of writing (15) are selected from chalk, pen and pencil.

In yet another embodiment the rulers (7) and (8) are retractable.

### General description

This application arises from the need for the existence of a tool that combines, in a single product, the instruments essential for assessing the risk of falling for older people living in the community, where, after applying the respective validity tests, it is possible to diagnose the risk of falling in this population by accurately identifying the people who did not fall, those who fell once and also those who fell more than once in the retrospective space and in the prospective space of one year, which is to say, one year before and one year after evaluation with this tool.
This technology also comprises the submission of data acquired using the instruments of the tool, and a dynamic model with a statistical basis and an Internet connection, makes it possible to determine with great accuracy the risk of falling in older people who live in the community.

The tool hereby described was developed from a trolley-style box, built specifically to comprise different measuring instruments that allow for the application of physical, functional and cognitive tests, and clinical questionnaires validated in the international literature, in order to obtain data to assess the risk of falling.

This multidimensional assessment of the risk of falling generates, using dynamic statistical modelling of the individual results of the various instruments used, a continuous result of the risk of falling with the respective cutoff values adjusted to the characteristics of the population.

Additionally, this technology allows for the regular screening of the risk of falling in older people, with great ease of implementation, which can be applied not only by health professionals, i.e., formal caregivers, but also by informal caregivers. The easy applicability and high precision of the tool hereby described represents a considerable advancement in the provision of primary health care, enabling the diagnosis of the risk of falling to considerable proportions of the population, which will allow for the development of specific and personalized preventive interventions for people with an increased risk of falling. It is also noteworthy that, due to the fact that the tool is presented in a trolley configuration, it allows for the easy transport of all the instruments, enabling the tool to be brought directly to the population for evaluation without the latter, which may suffer locomotive or other limitations, having to go to a specific assessment site.

This technology presents enormous commercial potential, serving both for clinical diagnosis in the services provided by formal health care services (health centers, hospitals, diagnostic clinics or rehabilitation clinics) as well as for scientific and pedagogical purposes (universities, health schools or research centers) and for private use (informal caregivers, older people or their relatives).

### Brief description of the figures

For an easier understanding of this application, figures are attached which represent preferential embodiments but which are not intended to limit the technique disclosed hereby.
Figure 1 illustrates the first level of instrumentation of the tool (1) for assessing the risk of falling, where the reference numbers represent the following instruments to be positioned in the locations indicated: 2 - digital stopwatches; 12 - bioimpedance scale; 13 - metronome;14 - mattresses; 16 - instructions manual and digital medium with manual.
Figure 2 illustrates the first level of instrumentation of the tool (1) for assessing the risk of falling and an increased zone A where the instruments are seen 2 - digital stopwatches and 13 - a metronome placed in the respective locations of the tool.
Figure 3 illustrates the second instrumentation level of the tool (1), where the reference numbers represent the following instruments to be placed in the cited locations: 3 - anthropometric tape; 4 - tape measure; 5 - flat markers; 6 - retractable cones; 7 - metal ruler; 8 - metal ruler; 9 - stadiometer; 10 - dumbbell weight; 11 - threaded dumbbell handle; 15 - means of writing.
Figure 4 illustrates the support (17) for the flat markers (5) and the retractable cones (6) that are illustrated in the retracted position and extended position.
Figure 5 illustrates the vertical layout that the plane markers (5) and the retractable cones (6) assume when arranged on the support (17) of Figure 4.

### Description of the embodiments

With reference to the figures, some embodiments are now described in more detail, but are not intended to limit the scope of this application.

This technology concerns a tool comprising several instruments for measuring the risk of falling in order people to obtain results from the application of physical, functional and cognitive tests to be submitted to a dynamic model with a statistical basis, that makes it possible to determine, with great accuracy, the risk of falling.

The way the tool is built allows not only for the secure and specific connection of each instrument, but also for the execution of certain tests to assess the risk of falling using the toolbox itself. The characteristics of the materials that constitute the toolbox and the geometric shape that it assumes, allows for the realization of tests to evaluate the strength of the lower limbs, agility and dynamic balance. The constituent material of the inside of the tool allows for the personalization of the instruments, permitting the elaboration of various combinations of the same instruments in its interior, depending on the typology of the tool desired, allowing the assessor to request a complete or partial tool in accordance with the characteristics that he intends to evaluate in the older people.

As the fall risk prediction model is a dynamic model, it allows for the adjustment of the final result of the risk in accordance with the tests performed.
This option provided by the tool is unique in a single product, and currently no other tool allows for this diversity in the multidimensional assessment of the risk of falls in older people, guaranteeing high levels of accuracy, reliability and applicability.

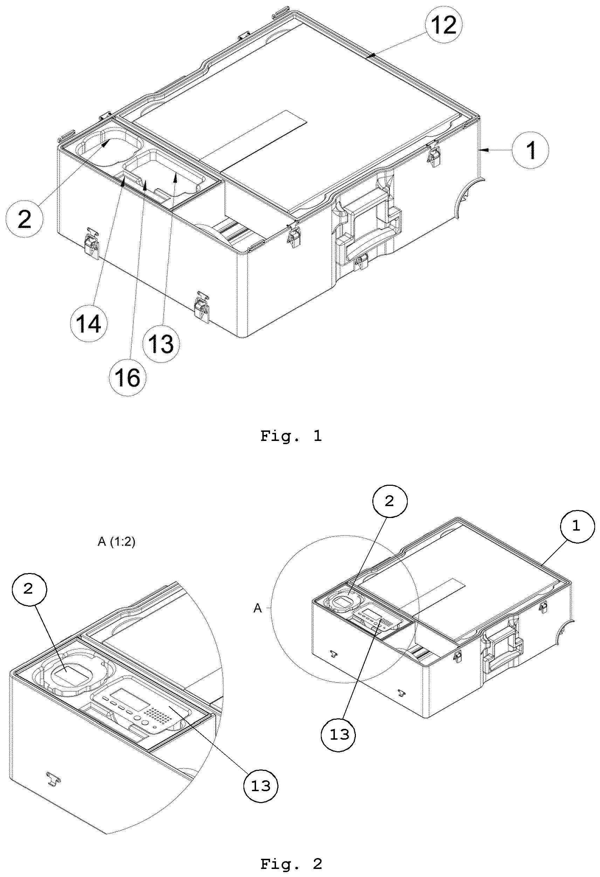

In accordance with figures 1, 2 and 3, the tool (1) consists of a trolley and is designed to duly accommodate the various measuring instruments that allow for the multidimensional assessment of the risk of falling.

The tool (1) thus comprises digital stopwatches (2), a 150cm anthropometric tape (3), a 20m measuring tape (4), flat markers (5), 30cm high retractable cones (6), a 30cm ruler (7), a 50cm ruler (8), a stadiometer (9), threaded dumbbell handles (11), threaded dumbbell weights (10), a bioimpedance scale (12), a metronome (13), mattresses (14), means of writing (15), an instruction manual for application and interpretation of the results and digital medium with the instruction manual (16), preferably a CD, with a tutorial for installation of the computer application via the Internet and an alternative dynamic model to bioimpedance.

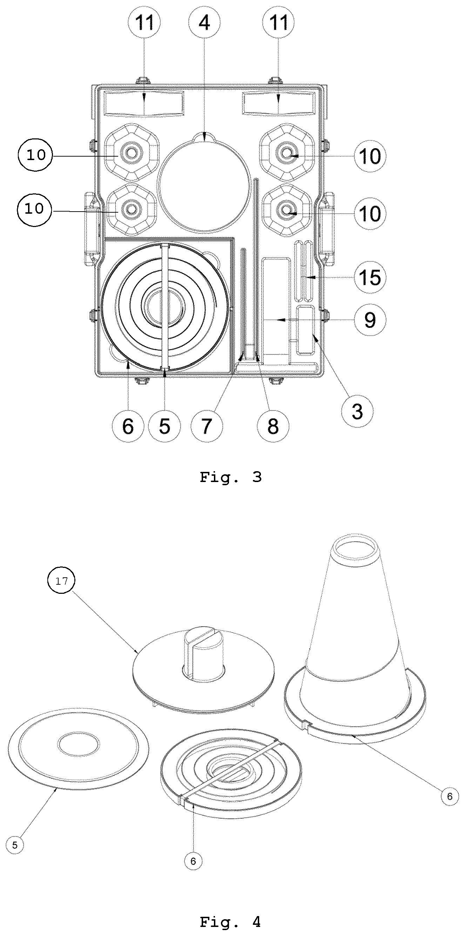

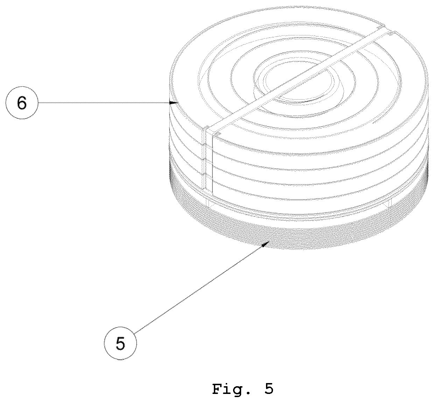

The various instruments are divided into two consecutive levels in the tool (1). In one embodiment on the first level, as shown in Figure 1 and 2, the following instruments are placed in the tool (1) : two digital stopwatches (2), the bioimpedance scale (12), the metronome (13), two mattresses (14), and the instruction manual and digital medium with the manual (16).
In a second level of the tool (1), below the instruments of the first level, as shown in Figure 3, the following instruments are placed in the tool (1) : 150cm anthropometric tape (3); the 20m Measuring tape (4); ten flat markers (5)(Figure 4); four 30cm high retractable cones (6)(Figure 4); a 30 cm ruler (7); a 50 cm ruler (8); a stadiometer (9); two threaded dumbbell handles (11); four threaded dumbbell weights (10); a means of writing (15).
In accordance with Figure 4, the tool (1) also includes a support (17) for the flat markers (5) and for the cones (6) in order that they can be vertically arranged as shown in Figure 5.

The digital stopwatches (2) allow for the measurement of time in the performance of the tests of agility, balance, strength, aerobic endurance and cognition in motor tasks. The anthropometric tape (3) allows for the measurement of the abdominal and hip circumferences of the subject to be evaluated, as measures of body composition.
The 20m measuring tape (4), the ten flat markers (5) and the four retractable cones (6) allow for the mounting of an aerobic endurance evaluation course using a 6-minute walking test.
The 30cm ruler (7) and the 50cm ruler (8) allow for the realization of the tests of balance and self-perception of limits of action.

The stadiometer (9) and the bioimpedance scale (12) allow for the evaluation of stature and body composition, respectively.
The threaded dumbbell handle (11), together with the threaded dumbbell weights (10), allow for the performance of the strength evaluation test of the upper limbs.
The metronome (13) allows for the realization of the dynamic balance test.
The existence of the two mattresses allows for the realization of the dynamic balance tests on unstable surfaces.

The instruction manual (16) for the application of the tests and interpretation of the results allows for the understanding of the protocols of the various tests and of the cutoff values for the risk of falling, in paper format. The digital medium with the instruction manual and installation tutorial for the computer application to guide the user both in the protocols of the tests and the cutoff values from a digital perspective, and with the installation of all the functions of the application that allows for the recording of the results and the obtaining of the risk developed specifically for this tool.
The digital medium with the manual also contains a tutorial that allows, through the computer application, for the substitution of the tests performed on the bioimpedance scale to assess body composition, given the existence of people with metal prostheses in whom bioimpedance does not work. Thus, an alternative dynamic model for bioimpedance was developed for this purpose, which combines the results of other variables of body composition to calculate the risk of falling.

The tool trolley box (1) is made from thermo-molded plastic material with features that give it strength and durability. In one embodiment, the toolbox (1) has dimensions of 150mm in height, 360mm in width and 460mm in length.

In one embodiment each threaded dumbbell handle (10) weighs 0.91 kg.
In another embodiment each threaded dumbbell weight (11) weighs 0.68 kg.
In one embodiment the ten flat markers (5) are made from semi-rigid, thermo-molded plastic material.
In another embodiment the support (17) for the flat markers (5) and retractable cones (6) is made from acrylonitrile butadiene styrene (ABS) material.
In one embodiment, the retractable cones (6), have their base made from ABS material, which can be produced by three-dimensional printing, two metal structural elements, and the body of the cone made from polyester material.
In one embodiment the 30cm ruler (7) is retractable and made of metallic material, preferably aluminum.
In another embodiment the 50cm ruler (8) is retractable and made of metallic material, preferably aluminum.
In one embodiment the threaded handle of the dumbbell (11) is made from metal and may be coated with silicon.
In one embodiment each of the threaded dumbbell weights (10) is made of metal and may be silicone-coated.
In one embodiment the means of writing (15) are selected from chalk, pen, pencil.
The reliability results performed for the tool (1) also presented good indices:
Intra-rater reliability: intraclass correlation = 0.95; inter-rater reliability: intraclass correlation = 0.89, regardless of whether the evaluator is a formal or informal caregiver, proving the high applicability of this technology.

### Tests

The tests to be performed in order to assess the risk of falling of an older subject are now described demonstrating the use of each instrument during the various tests.

### Test of perception of affordances

Objective: This test allows for the assessment of the self-perception of the subject as to his maximum capacity to accomplish the task of overcoming a horizontal obstacle with one stride.
Instruments: This test uses the means of writing (15), a 50cm ruler (8) and a 20m measuring tape (4).

For this test it is necessary to draw a line on the ground with the means of writing (5) and the ruler (8). The ruler (8) is placed at a certain distance, which can be adjusted according to the subject, and parallel to the line drawn. The subject is placed behind the drawn line and must clear the predetermined distance in one step so as to completely pass over the ruler (8) that was placed on the ground. The distance covered by the stride is measured and recorded with the measuring tape (4) from the line drawn to the edge of the ruler (8).

### Battery of Fullerton Advanced Balance (FAB) scale tests

Objective: These tests assess dynamic and static balance, comprising ten tests quoted on an ordinal scale varying from 0 (worst) to 4 points (best). The result of the sum of all the tests corresponds to the multidimensional balance value. Instruments: These tests use a stopwatch (2) a 30cm ruler (7) a 50cm ruler (8), the tool box (1), the means of writing (15) to mark the ground, two mattresses (14) and one metronome (13).
- The test of remaining standing with one's feet together and eyes closed assesses the ability to use proprioceptive information to keep one's balance in a standing position, with a reduced support base and without visual stimuli, where the stopwatch (2) measures the duration of the test;
- The test of reaching an object with a lean on the frontal plane without altering the support base evaluates the ability to perform a forward lean to reach an object by evaluating the limits of stability on the frontal plane. This test uses the means of writing (15) and the 30cm ruler (7) ruler is used to place the object (writing medium (15)) at a horizontal distance of 25 cm from the participant's hand when its arm is extended horizontally and at an angle of 90° from the body;
- The test of clearing a step assesses the control of the center of gravity, and measures the lower body strength and the bilateral motor coordination. The toolbox (1) is used in this test as the step to be cleared where the subject must first climb the step with one foot and subsequently completely clear the step with the other foot;
- The ten-step walk in a line evaluates the dynamic control capacity of the center of mass with an altered support base. A writing medium (15) is used to mark the ground with a distance that allows the participant to take ten steps along the mark;
- The unipedal balance test evaluates the maintenance of balance in an upright position supported only on one leg, where the stopwatch (2) counts the time of the duration of the test;
- The test of standing on a semi-rigid surface with one's eyes closed assesses the ability to maintain one's balance in an upright position using the mattresses (14) as a semi-rigid surface and the stopwatch (2) to measure the duration of the test;
- The two-foot jump test assesses the coordination capacity of the upper and lower body, and the lower body power, using a flat marker (5) placed on the ground, and the 50cm ruler (8) to measure a distance of twice the length of the foot of the subject from the marker (5), marking the maximum distance with the ruler (8) or the means of writing (15). The subject must jump with its feet together from the marker (5) in order to clear the distance marked;
- The gait test with rotation of the head evaluates the ability to maintain dynamic balance during a ten-step walk while turning one's head, using the metronome (13) at 100 beats per minute for the subject to rotate its head to the rhythm of the metronome (13) during the walk.

### Evaluation of body composition

Objective: The body composition tests evaluate fat mass, lean mass, basal metabolism and visceral fat.
Instruments: These tests use a stadiometer (9), a bioimpedance scale (12), and an anthropometrical tape (3).
- The measurement using bioimpedance evaluates the fat mass (%), lean mass (%), basal metabolism and visceral fat, using the bioimpedance scale;
- The height and weight measurement determine the body mass index (KG/M²), using the bioimpedance scale (12) and the stadiometer (9);
- The measurement of the circumference of the waist and hip is performed using the anthropometric tape (3).

### Adjusted battery of Senior Fitness Tests (SFT)

Objective: This battery comprises eight tests assessing functional fitness in order to evaluate lower and upper strength, body composition, lower and upper flexibility, agility, cardiorespiratory capacity, and the ability to perform dual motor and cognitive tasks.
Instruments: These tests use a stopwatch (2), a 2.27kg dumbbell formed of the dumbbell handle (11) and two weights (10)) to perform the test with female subjects, a 3.63kg dumbbell formed of the dumbbell handle (11) and four weights (10) to perform the test with male subjects, five flat markers (5), four retractable cones 30cm in height (6), a 50cm ruler (8) and a 20m measuring tape (4).
- The test of getting up and sitting down on a chair assesses the strength and resistance of the lower limbs and uses a stopwatch (2) to measure the duration of the test;
- The forearm flexion test assesses the strength and endurance of the upper limbs and uses the stopwatch (2) to measure the duration of the test and the weights with the aim that the subject performs the maximum number of flexions with the arm holding the weights;
- The test of reaching while seated assesses the flexibility of the trunk and the lower limbs of the subject, using the 50cm ruler (8), to measure the distance the subject can reach towards his foot with his leg extended;
- The test of reaching behind the back assesses the flexibility of the upper limbs of the subject, where one hand is placed above the corresponding shoulder, while the other hand is placed behind the back in the lumbar region, with the two hands trying to reach each other. The 50cm ruler (8) is used to measure the distance between the middle fingers of both hands;
- The test of sitting, walking 2.44m and sitting back down again assesses the physical mobility, speed, agility and dynamic balance of the subject, where a cone (6) is placed at a distance of 2.44m in front of the subject seated in a chair, measured with the measuring tape (4). The subject must cover the distance to the cone (6) and back to the chair, with the duration of the test being measured with the stopwatch (2);
- The double task test is similar to the previous test, but the subject must perform a cognitive task of counting backwards while performing the test. This test can be adapted using other cognitive tasks;
The six-minute walk test evaluates aerobic endurance, using a rectangular route with a 50m perimeter, 20m in length and 5m in width. The four corners of the rectangle are marked with the 30cm high retractable cones (6) and flat markers are placed every 5m (5), using the 20m measuring tape for the markings (4), a marking for the start of the course, and the stopwatch (2) to measure the duration of the test.

This multidimensional assessment of the risk of falls, based on a dynamic statistical modelling of the individual results of the various instruments used, generates a continuous result of the risk of falls with their respective cutoff values adjusted to the characteristics of the population.

This statistical modelling involves four successive steps:
1. all the variables are normalized (log¹⁰);
2. a Principal Component Analysis (PCA) with orthogonal rotation (varimax) is performed, and subsequently, the variables with a *loading factor* greater than or equal to 0.4, which share at least 15% of the variance with the factor, are used for the constitution of the principle components. This analysis is carried out separately for men and women, and by age groups, specifically for the groups: ≥ 65 years to < 75 years, ≥ 75 years to < 85 years, ≥ 85 years to < 95 years and ≥ 95 years;
3. the result of the risk of continuous falls is calculated by the sum of the individual records of the principal components, each weighted for the relative contribution of the principal components for the variance explained.
4. the cutoff values for the continuous risk of falls are calculated specifically for each population, using an ROC (Receiving Operating Characteristic) analysis, commonly used to assess the performance of any continuous variable to discriminate between two mutually exclusive states of illness or risk. The specificity and sensitivity measures generated by the ROC analysis enable the determination of the value that identifies the people, of different genders and age groups, at high risk of falling.

The present description is not, of course, in any way restricted to the embodiments presented in this document and a person with average knowledge of the field may foresee many possibilities for modifying it without deviating from the general idea, as defined in the claims. The preferred embodiments described above can obviously be combined with each other. The following claims additionally define preferential embodiments.

## Claims

1. Multidimensional tool (1) for assessing the risk of falling in older people consisting of a trolley comprising two consecutive levels of instruments wherein:
- a first level of the two consecutive levels of the multidimensional tool (1) comprises digital stopwatches (2), a bioimpedance scale (12), a metronome (13), mattresses (14), a digital medium with an instruction manual (16) and installation tutorial for a computer application, and with an installation of all the functions of the computer application that allows for the recording of results and obtaining of a risk developed specifically for the multidimensional tool, and a tutorial that allows, through the computer application, for a substitution of tests performed on the bioimpedance scale to assess body composition, and an alternative dynamic model for bioimpedance which combines results of other variables of body composition to calculate the risk of falling;
- a second of the two consecutive levels of the multidimensional tool (1) comprises a 150cm anthropometric tape (3), a 20m measuring tape (4), flat markers (5), retractable cones 30cm in height (6), a 30cm ruler (7), a 50cm ruler (8), a stadiometer (9), threaded dumbbell handles (11), dumbbell weights (10), and writing media (15); wherein the multidimensional tool (1) is suitable for the realization of tests to evaluate the strength of the lower limbs, agility and dynamic balance; wherein the multidimensional tool (1) is made from thermo-molded plastic material and has the dimensions of 150mm in height, 360mm in width and 460mm in length.

2. Multidimensional tool (1) according to any of the previous claims comprising four retractable cones (6).

3. Multidimensional tool (1) according to any of the previous claims comprising four dumbbell weights (10).

4. Multidimensional tool (1) according to any of the previous claims comprising two dumbbell handles (11).

5. Multidimensional tool (1) according to any of the previous claims wherein each dumbbell weight (10) weighs 0.68 Kg.

6. Multidimensional tool (1) according to any of the previous claims wherein each dumbbell handle (11) weighs 0.91 Kg.

7. Multidimensional tool (1) according to any of the previous claims comprising ten flat markers (5).

8. Multidimensional tool (1) according to any of the previous claims comprising two digital stopwatches (2).

9. Multidimensional tool (1) according to the previous claims comprising a support (17) for the flat markers (5) and the retractable cones (6).

10. Multidimensional tool (1) according to any of the previous claims comprising two mattresses (14).

11. Multidimensional tool (1) according to any of the previous claims wherein the means of writing (15) are selected from either chalk, pen, pencil.

12. Multidimensional tool (1) according to any of the previous claims wherein the rulers (7) and (8) are retractable.

## Patentansprüche

1. Mehrdimensionales Werkzeug (1) zur Beurteilung des Fallrisikos in älteren Personen, bestehend aus einem Wagen, der zwei aufeinander folgende Stufen von Instrumenten aufweist, worin:
- eine erste Stufe der beiden aufeinander folgenden Stufen des mehrdimensionalen Werkzeugs (1) digitale Stoppuhren (2), eine Bioimpedanzwaage (12), ein Metronom (13), Matratzen (14), ein digitales Medium mit einer Bedienungsanleitung (16) und einer Installationsanleitung für eine Computeranwendung und der Installation aller Funktionen der Computeranwendung aufweist, die die Aufzeichnung der Ergebnisse und den Erhalt eines Fallrisikos ermöglicht, das speziell für das mehrdimensionale Werkzeug entwickelt wurde und einer Anweisung, die eine Substitution der mit der Bioimpedanzwaage ausgeführten Tests durch die Computeranwendung ermöglicht, um die Körperzusammensetzung zu beurteilen, sowie ein alternatives dynamisches Modell für die Bioimpedanz, welches die Ergebnisse anderer Variablen der Körperzusammensetzung vereint, um das Fallrisiko zu berechnen;
- eine zweite der beiden aufeinander folgenden Stufen des mehrdimensionalen Werkzeugs (1) aus einem 150 cm anthropometrischem Maßband (3), einem 20 m Maßband (4), Flachmarkierungen (5), versenkbaren Kegeln mit 30 cm Höhe (6), einem 30 cm Lineal (7), einem 50 cm Lineal (8), einem Stadiometer (9), Hantelgriffen mit Gewinde (11), Hantelgewichten (10) und Schreibmitteln (15) besteht;
worin das mehrdimensionale Werkzeug (1) zur Durchführung von Tests geeignet ist, um die Stärke der unteren Gliedmaßen, die Beweglichkeit und das dynamische Gleichgewicht zu beurteilen;
worin das mehrdimensionale Werkzeug (1) aus thermogeformten Kunststoffmaterial hergestellt ist und eine Höhe von 150 mm, eine Breite von 360 mm und eine Länge von 460 mm aufweist.

2. Mehrdimensionales Werkzeug (1) gemäß einem der vorstehenden Ansprüche, bestehend aus vier versenkbaren Kegeln (6).

3. Mehrdimensionales Werkzeug (1) gemäß einem der vorstehenden Ansprüche, bestehend aus vier Hantelgewichten (10) .

4. Mehrdimensionales Werkzeug (1) gemäß einem der vorstehenden Ansprüche, bestehend aus zwei Hantelgriffen (11) .

5. Mehrdimensionales Werkzeug (1) gemäß einem der vorstehenden Ansprüche, worin jedes Hantelgewicht (10) 0,68 kg wiegt.

6. Mehrdimensionales Werkzeug (1) gemäß einem der vorstehenden Ansprüche, worin jeder Hantelgriff (11) 0,91 kg wiegt.

7. Mehrdimensionales Werkzeug (1) gemäß einem der vorstehenden Ansprüche, bestehend aus zehn Flachmarkierungen (5).

8. Mehrdimensionales Werkzeug (1) gemäß einem der vorstehenden Ansprüche, bestehend aus zwei digitalen Stoppuhren (2).

9. Mehrdimensionales Werkzeug (1) gemäß einem der vorstehenden Ansprüche, bestehend aus einem Halter (17) für die Flachmarkierungen (5) und die versenkbaren Kegel (6) .

10. Mehrdimensionales Werkzeug (1) gemäß einem der vorstehenden Ansprüche, bestehend aus zwei Matratzen (14).

11. Mehrdimensionales Werkzeug (1) gemäß einem der vorstehenden Ansprüche, worin die Schreibmittel (15) entweder aus Kreide, Schreibstiften oder Bleistiften gewählt werden.

12. Mehrdimensionales Werkzeug (1) gemäß einem der vorstehenden Ansprüche, worin die Lineale (7) und (8) einklappbar sind.

## Revendications

1. Outil multidimensionnel (1) pour l'évaluation du risque de chute chez des personnes âgées, constitué d'un chariot comportant deux niveaux consécutifs d'instruments dans lequel :
- un premier niveau parmi les deux niveaux consécutifs de l'outil multidimensionnel (1) comprend des chronomètres numériques (2), une balance impédancemètre (12), un métronome (13), des coussins (14), un support numérique avec un manuel d'instructions (16) et un tutoriel d'installation pour une application informatique, et avec une installation de toutes les fonctions de l'application informatique qui permettent l'enregistrement de résultats et l'obtention d'un risque développé spécifiquement pour l'outil multidimensionnel, et un tutoriel qui permet, via l'application informatique, une substitution de tests effectués sur la balance impédancemètre pour évaluer la composition corporelle, et un modèle dynamique de bioimpédance alternatif qui combine des résultats d'autres variables de composition corporelle pour calculer le risque de chute ;
- un deuxième niveau parmi les deux niveaux consécutifs de l'outil multidimensionnel (1) comprend un ruban anthropométrique de 150 cm (3), un mètre ruban de 20 m (4), des marqueurs plats (5), des cônes rétractables de 30 cm de hauteur (6), une règle de 30 cm (7), une règle de 50 cm (8), un stadiomètre (9), des poignées d'haltères filetées (11), des poids d'haltères (10) et des supports d'écriture (15) ;
dans lequel l'outil multidimensionnel (1) est adapté pour la réalisation de tests afin d'évaluer la résistance des membres inférieurs, l'agilité et l'équilibre dynamique ;
dans lequel l'outil multidimensionnel (1) est fabriqué à partir de matière plastique thermo-moulée et a des dimensions de 150 mm de haut, 360mm de large et 460mm de long.

2. Outil multidimensionnel (1) selon l'une quelconque des revendications précédentes comprenant quatre cônes rétractables (6).

3. Outil multidimensionnel (1) selon l'une quelconque des revendications précédentes comprenant quatre poids d'haltère (10) .

4. Outil multidimensionnel (1) selon l'une quelconque des revendications précédentes comprenant deux poignées d'haltère (11).

5. Outil multidimensionnel (1) selon l'une quelconque des revendications précédentes dans lequel chaque poids d'haltère (10) pèse 0,68 Kg.

6. Outil multidimensionnel (1) selon l'une quelconque des revendications précédentes dans lequel chaque poignée d'haltère (11) pèse 0,91 Kg.

7. Outil multidimensionnel (1) selon l'une quelconque des revendications précédentes comprenant dix marqueurs plats (5) .

8. Outil multidimensionnel (1) selon l'une quelconque des revendications précédentes comprenant deux chronomètres numériques (2).

9. Outil multidimensionnel (1) selon l'une quelconque des revendications précédentes comprenant un support (17) pour les marqueurs plats (5) et les cônes rétractables (6)

10. Outil multidimensionnel (1) selon l'une quelconque des revendications précédentes comprenant deux coussins (14).

11. Outil multidimensionnel (1) selon l'une quelconque des revendications précédentes dans lequel les moyens d'écriture (15) sont sélectionnés parmi soit une craie, un stylo, un crayon.

12. Outil multidimensionnel (1) selon l'une quelconque des revendications précédentes dans lequel les règles (7) et (8) sont rétractables.
